# EUROPEAN PATENT SPECIFICATION

(11) **EP 2 473 153 B1**
(45) Date of publication and mention of the grant of the patent: **25.10.2017**
(21) Application number: 09792103.5
(22) Date of filing: 31.08.2009
(51) Int. Cl.: A61Q 19/10, A61K 8/73, A61K 8/02

(54) **SURFACE MODIFIED PIGMENT**
OBERFLÄCHENMODIFIZIERTES PIGMENT
PIGMENT MODIFIÉ EN SURFACE

(43) Date of publication of application: 11.07.2012
(73) Proprietor: Colgate-Palmolive Company, New York, NY 10022 (US); Kobo Products Inc., South Plainfield, NJ 07080 (US)
(72) Inventor: SHAO, Yun, Belle Mead, NJ 07080 (US); SCHLOSSMAN, David, Short Hills, NJ 07078 (US); KONTRIMIENE, Neringa, Somerset, NJ 08873 (US); HASKEL, Ariel, East Brunswick, NJ 08816 (US)
(74) Representative: Wichmann, Hendrik
(86) International application number: PCT/US2009/055510
(87) International publication number: WO 2011/025503

(56) References cited:
- WO-A1-2004/100921
- WO-A1-2007/101586
- WO-A2-00/12053
- JP-A- 2004 203 788
- US-A- 6 083 491
- US-B1- 6 432 417
- S. L. Jordan et al.: "Effect of Hydrophobic Substitution on Cationic Conditioning Polymers"; "Chapter 2" In: Sarah E. Morgan, Kathleen O. Havelka, Robert Y. Lochhead: "Cosmetic Nanotechnology - Polymers and Colloids in Cosmetics" 12 April 2007 (2007-04-12), , XP9137227 vol. 961, , pages 59-71 page 67 - page 69
- "Polyquaternium-67" In: TARA E GOTTSCHALCK ET AL: "International Cosmetic Ingredient, Dictionary and Handbook" 2008, , XP002595503 vol. 2, , page 2132 * abstract

## Description

### BACKGROUND OF THE INVENTION

There is a desire to increase the radiance of skin in such a way that consumers can perceive their skin being radiant, glossy, and/or shiny. These attributes are evaluated via consumer assessment. Skin radiance is also correlated to skin gloss measurements. While radiance can be imparted to skin by using a leave on product that deposits a pigment that can increase gloss, it would be desirable to be able to increase gloss by using a cleansing composition that includes a pigment.

The problem with using a cleansing composition is that the composition performs its function of cleaning by removing oil and dirt from skin. To be effective, the pigment needs to be modified to adhere to skin to avoid being washed away by the cleansing composition.

It is desirable to create a pigment that can adhere to skin that can be delivered from a cleansing composition.

WO-A-2007/101586 discloses hydrophilic structured bar compositions comprising individually coated flat platy particles, each having surface deposition chemistry mechanism. WO-A-2004/100921 discloses a method for depositing shiny particles on keratinous surfaces from rinse-off compositions. US-A-6,083,491 discloses cosmetic compositions containing a dispersion of solid particles, the surface of which is coated with a cationic polymer. WO-A-00/12053 discloses makeup compositions and methods of making same. JP-A-2004/203788 discloses solid water-in-oil type emulsified cosmetics. US-A-2009/0162408 discloses compositions containing cationically surface-modified microparticulate carrier for benefit-agents.

### SUMMARY OF THE INVENTION

The present invention provides a cleansing composition according to claim 1, the composition comprising a pigment particle that is pre-coated with Polyquaternium-67. The present invention also provides a method according to claim 9 of increasing gloss on a substrate comprising applying the composition to the substrate. Preferred features are defined in the dependent claims.

### DETAILED DESCRIPTION

As used throughout, ranges are used as a shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The present disclosure relates to a pigment particle that is pre-coated with Polyquaternium-67.

The pigment particle imparts gloss and is selected from mica/titanium dioxide, mica/iron oxide, mica, and combinations thereof. In one embodiment, the pigment particle is mica/titanium dioxide. The particle size can be chosen to be any size that provides a measurable gloss. The particle size of the particle is the size before coating with the cationic material or any other coating. In one embodiment, the particle size is 1 to 1000 microns. In other embodiments, the particle size is at least 10, 20, 30, 40, 50, 60, 60, 80, 90, 100, or 150 microns. In other embodiments, the particle size is up to 100, 150, 250, 300, 350, 400, 450, 500, 600, 700, 800, 900, or 1000 microns. Any of the preceding minimum amounts can be combined with any of the maximum amounts to form a range. In one embodiment, the particle size is 50 to 100 microns.

Polyquaternium-67 is the generic INCI name for a polymeric quaternary ammonium salt of hydroxyethyl cellulose reacted with a trimethyl ammonium substituted epoxide and a lauryl dimethyl ammonium (dimethyldodecyl) substituted epoxide. Polyquaternium-67 is available from the Amerchol subsidiary of Dow Chemical Company under the SoftCAT trade name. In one embodiment, the Polyquaternium-67 is SoftCAT SK-MH.

The amount of Polyquaternium-67 on the total weight of the pigment particle is 0.1 to 5 weight %. In other embodiments the amount of cationic material is at least 0.1, 0.2, 0.3, 0.4, 0.5, 0.6, 0.7, 0.8, 0.9, 1, 1.5, 2, 2.5, 3, 3.5, 4, or 4.5 weight %. In other embodiments, the amount of cationic material is less than 1, 1.5, 2, 2.5, 3, 3.5, 4, 4.5, or 5 weight %. Any of the preceding minimum amounts can be combined with any of the maximum amounts to form a range. In another embodiment, the pigment particle has a particle size of 50 to 100 microns, and the amount of cationic material on these particles can be any of the preceding amounts.

The pigment particle is pre-coated. By pre-coated, it is meant that the pigment particle is coated with the cationic material prior to the pigment particle being added to a composition that contains a material in addition to itself.

In another embodiment, the pigment particle can further include isopropyl titanium triisostearate (ITT). The isopropyl titanium triisostearate can be added to the pigment particle before or after the cationic material is applied to the pigment particle. In one embodiment, the amount of ITT on the total weight of the pigment particle is 0.1 to 5 weight%. In other embodiments, the amount of ITT is at least 0.1, 0.5, 1, 1.5, 2, 2.5, 3, 3.5, 4, or 4.5 weight%. In other embodiments, the amount of ITT is less than 1, 2, 3, 4, or 5 weight %. Any of the preceding minimum amounts can be combined with any of the maximum amounts to form a range. In another embodiment, the pigment particle has a particle size of 50 to 100 microns, and the amount of ITT on these particles can be any of the preceding amounts.

A pigment particle with the Polyquaternium-67 can be made using the following procedure.
(i) The Polyquaternium-67 is added under mixing to water in a vessel in which there is a sufficient amount of the water to dissolve the Polyquaternium-67 under heating.
(ii) The Polyquaternium-67/water is heated and mixed until the Polyquaternium-67 is dissolved.
(iii) Pigment is mixed into the mixture.
(iv) The mixture is dried at 105°C until sufficiently dry (approximately 3 hours).
(v) The material is cooled and chopped gently.
(vi) The material is sieved (for example in a No. 40 sieve) to remove agglomerate.

A particle with the Polyquaternium-67 that includes the ITT added after the Polyquaternium-67 is applied can be made using the following procedure.
(i) The Polyquaternium-67 is added under mixing to water in a vessel in which there is a sufficient amount of the water to dissolve the Polyquaternium-67 under heating.
(ii) The Polyquaternium-67/water is heated and mixed until the Polyquaternium-67 is dissolved.
(iii) Pigment is mixed into the mixture.
(iv) The mixture is dried at 105°C until sufficiently dry (approximately 3 hours).
(v) The material is cooled and chopped gently.
(vi) The material is sieved (for example in a No. 40 sieve) to remove agglomerate.
(vii) Isopropyl titanium triisostearate is dissolved in ISOPAR C solvent in a ratio of 1:2.
(viii) The solution is added to the powder obtained in step (ix) under mixing in a blender.
(ix) The solution is dried at 85°C for 1 hour.
(x) The material is cooled and chopped gently.

A particle with the Polyquaternium-67 that includes the ITT added before the Polyquaternium-67 is applied can be made using the following procedure.
(i) Isopropyl titanium triisostearate is dissolved in ISOPAR C solvent in a ratio of 1:2.
(ii) The solution is added to pigment particles under mixing in a blender.
(iii) The solution is dried at 85°C for 1 hour.
(iv) The material is cooled and chopped gently.
(v) The Polyquaternium-67 is added under mixing to water in a vessel in which there is a sufficient amount of the water to dissolve the Polyquaternium-67 under heating.
(vi) The Polyquaternium-67/water is heated and mixed until the Polyquaternium-67 is dissolved.
(vii) Pigment from step (iv) is mixed into the mixture.
(viii) The mixture is dried at 105°C until sufficiently dry (approximately 3 hours).
(ix) The material is cooled and chopped gently.
(x) The material is sieved (for example in a No. 40 sieve) to remove agglomerate.

The pigment particle can be included in a cleansing composition, such as a body wash, shower gel, liquid hand soap, or bar soap. The amount of the particle in the cleansing system can be any amount that is generally used for particles. In certain embodiments, the amount is 0.01 to 20 weight % of the composition, 0.1 to 10 weight %, or 0.15 to 2 weight %. In other embodiments, the amount is at least 0.1, 0.15, 0.2, 0.3, 0.4, 0.5, 0.6, 0.7, 0.8, 0.9, 1, 1.2, 1.2, 1.3, 1.4, 1.5, or 2 weight % up to 20 weight %. The particle can be added to the cleansing composition by any standard addition procedure for adding pigments.

A variety of anionic surfactants can be utilized in a cleansing composition including, for example, long chain alkyl (C₆-C₂₂) materials such as long chain alkyl sulfates, long chain alkyl sulfonates, long chain alkyl phosphates, long chain alkyl ether sulfates, long chain alkyl alpha olefin sulfonates, long chain alkyl taurates, long chain alkyl isethionates (SCI), long chain alkyl glyceryl ether sulfonates (AGES), sulfosuccinates, Stepan-Mild™ PCL sodium methyl-2 sulfolaurate and disodium 2-sulfolaurate and sodium lauryl sulfoacetate blend, and the like. These anionic surfactants can be alkoxylated, for example, ethoxylated, although alkoxylation is not required. These surfactants are typically highly water soluble as their sodium, potassium, alkyl and ammonium or alkanol ammonium containing salt form and can provide high foaming cleansing power. Other equivalent anionic surfactants may be used. In one embodiment, the anionic surfactant comprises sodium laureth sulfate, sodium pareth sulfate, and combinations thereof. Anionic surfactants can be included in any desired amount. In one embodiment, anionic surfactants are present in the composition in an amount of 0 to about 15% by weight. In one embodiment, anionic surfactants are present in an amount of about 6 to about 8% by weight.

Amphoteric surfactants may also be included in the composition. These surfactants are typically characterized by a combination of high surfactant activity, lather forming and mildness. Amphoteric surfactants include, but are not limited to, derivatives of aliphatic secondary and tertiary amines in which the aliphatic radical can be straight chain or branched and wherein one of the aliphatic substituents contains about 8 to about 18 carbon atoms and one contains an anionic water solubilizing group, e.g., carboxy, sulfonate, sulfate, phosphate, or phosphonate. Examples of such compounds include sodium 3-dodecyaminopropionate, sodium 3-dodecylaminopropane sulfonate, N-alkyl taurines and N-higher alkyl aspartic acids. Other equivalent amphoteric surfactants may be used. Examples of amphoteric surfactants include, but are not limited to, a range of betaines including, for example, high alkyl betaines, such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, sulfobetaines such as coco dimethyl sulfopropyl betaine, stearyl dimethyl sulfopropyl betaine, amido betaines, amidosulfobetaines and the like. Betaines having a long chain alkyl group, particularly coco, may be particularly useful as are those that include an amido groups such as the cocamidopropyl and cocoamidoethyl betaines. Amphoteric surfactants can be included in any desired amount. In one embodiment, amphoteric surfactants are present in the composition in an amount of 0 to about 15% by weight. In one embodiment, the amphoteric surfactants are present in the composition in an amount of about 4 to about 6% by weight.

Examples of nonionic surfactants include, but are not limited to, polysorbate 20, long chain alkyl glucosides having C₈-C₂₂ alkyl groups; coconut fatty acid monoethanolamides such as cocamide MEA; coconut fatty acid diethanolamides, fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acylpolyethylene glycols); polypropylene glycol ethoxylates (for example the PLURONIC™ block copolymers commercially available from BASF); fatty acid alkylolamides, (fatty acid amide polyethylene glycols); N-alkyl-, N-alkoxypolyhydroxy fatty acid amides; sucrose esters; sorbitol esters; polyglycol ethers; and combinations thereof. Nonionic surfactants can be included in any desired amount. In one embodiment, nonionic surfactants are present in the composition in an amount of 0 to about 3% by weight. In one embodiment, nonionic surfactants are present in the composition in an amount of about 0.5 to about 1.5% by weight.

Cationic surfactants can also be included in the composition. Examples of cationic. surfactants include, but are not limited to any quaternium or polyquaternium compound. Cationic surfactants can be included at any desired level. In one embodiment, cationic surfactants are present in the composition in an amount of 0 to about 2 % by weight. In one embodiment, cationic surfactants are present in the composition in an amount of about 0.1 to about 0.3 % by weight.

Many additional surfactants are described in McCUTCHEON'S DETERGENTS AND EMULSIFIERS (1989) and other reference materials that are well known to those of ordinary skill in the art.

Additionally, a suspending agent can be included to structure the surfactant to aid in suspending particles. Suspending agents are any material that increases the ability of the composition to suspend material. Examples of suspending agents include, but are not limited to, synthetic structuring agents, polymeric gums, polysaccharides, pectin, alginate, arabinogalactan, carrageen, gellan gum, xanthum gum, guar gum, rhamsan gum, furcellaran gum, and other natural gum. A synthetic structuring agent in one embodiment is a polyacrylate. One acrylate aqueous solution used to form a stable suspension of the solid particles is manufactured by Lubrizol as CARBOPOL™ resins, also known as CARBOMER™, which are hydrophilic high molecular weight, crosslinked acrylic acid polymers. In one embodiment, the polymer is CARBOPOL™ Aqua SF-1. Other polymers that can be used include, but are not limited to, CARBOPOL™ Aqua 30, CARBOPOL™ 940 with a molecular weight of approximately 4,000,000, and CARBOPOL™ 934 with a molecular weight of approximately 3,000,000.

The suspending agents can be used alone or in combination. The amount of suspending agent can be any amount that provides for a desired level of suspending ability. In one embodiment, the suspending agent is present in an amount of about 0.01 to about 15% by weight of the composition. In other embodiments, the amount of suspending agent is about 1% to about 10%.

The cleansing composition can contain an oil and/or petrolatum. Oils that can be used can be any vegetable oil, such as sunflower, soybean, castor, etc.

The pigment particle can also be included in an oral care composition for delivery of the pigment particle to a surface in the oral cavity, such as teeth.

The pigment particle can also be included in a home care composition, such as a hard surface cleaner, a dishwashing composition, a fabric softener, or a laundry detergent.

The composition can be used in a method to increase the gloss on a substrate. The composition is applied to the substrate. The particle that is used is a particle that can provide gloss. After applying, the composition can be left for a period of time and then removed. The period of time can be any desired period of time, such as 5, 10, 15, 20, 30, 45, or 60 seconds, or 1, 2, 3, 4, 5, 10, 15, 30, 60 minutes, or any length of time.

The applying can be from a cleansing composition in which the composition is applied to skin to wash the skin. After applying and lathering the composition on skin, the composition can be left for a period of time on skin before the lather is rinsed and the skin is dried.

### SPECIFIC EMBODIMENTS OF THE INVENTION

The invention is further described in the following examples. The examples are merely illustrative and do not in any way limit the scope of the invention as described and claimed.

Examples of making the pigment particles.

0.5 g of SoftCAT SK-MH is added to 50 g of distilled water in a beaker. It is heated and mixed until the SoftCAT SK-MH is totally dissolved. The solution is added to 98 g of KTZ® Shimmer White mica/titanium dioxide powder under mixing in a blender. After the addition is complete, the mixture is dried at 105°C for 3 h. It is cooled and chopped gently in a blender. The powder is sieved through a No. 40 sieve to remove agglomerate.

2g of isopropyl titanium triisostearate is dissolved in 4 g of Isopar C solvent. The solution is added dropwise to 100 g of the powder made in [0031] in a blender under mixing. After the addition is complete, the mixture is dried at 85°C for 1h. It is cooled and chopped gently in a blender to remove agglomerate.

2g of isopropyl titanium triisostearate is dissolved in 4 g of Isopar C solvent. The solution is added dropwise to 100 g af KTZ® Shimmer White mica/titanium dioxide powder in a blender under mixing. After the addition is complete, the mixture is dried at 85 C for 1 hour. It is cooled and chopped gently in a blender to remove agglomerate. This material can then be used in place of the KTZ® Shimmer White mica/titanium dioxide powder in paragraph [0031].

The base composition below is used to make body wash compositions described below. It is made by mixing the ingredients.

| Material | Weight % (active) |
|---|---|
| Sodium salt of C10-16 alcohol ethoxylate sulfate (2EO average) | 9.5 |
| Carbopol™ Aqua SF-1 acrylate thickening polymer | 2.7 |
| Cocamidopropyl betaine | 1.7 |
| Water, preservatives, and minors | Q.S. |

Three particles were tested against a control body wash without pigment and a pigment without treatment to measure the gloss on skin after washing with the body wash. The formulas are in the table below. Formula A contains a mica/titanium dioxide particle that is coated with polyquaternium-67. Formula B contains a mica/titanium dioxide particle that is first coated with polyquaternium-67 and then coated with isopropyl titanium triisostearate. Formula C contains a mica/titanium dioxide particle that is first coated with isopropyl titanium triisostearate and then coated with polyquaternium-67. The body washes are prepared by mixing the ingredients. In the table below, the amounts are based on the overall weight of the material.

| Material | Control | Untreated | A | B | C |
|---|---|---|---|---|---|
| Base | 96.99 | 96.19 | 96.19 | 96.19 | 96.19 |
| Mica/titanium dioxide pigment with no treatment | 0 | 0.8 | 0 | 0 | 0 |
| Pigment A | 0 | 0 | 0.8 | 0 | 0 |
| Pigment B | 0 | 0 | 0 | 0.8 | 0 |
| Pigment C | 0 | 0 | 0 | 0 | 0.8 |
| Perfume | 1 | 1 | 1 | 1 | 1 |
| 25 weight% salt solution | 2 | 2 | 2 | 2 | 2 |
| 50 weight % citric acid solution | 0.01 | 0.01 | 0.01 | 0.01 | 0.01 |

The body washes are tested on forearms of panelists. The forearms are prepared by:
(i) Wetting forearm under warm running water,
(ii) Pre-washing the forearm with Palmolive bar soap (green variant) from Colgate-Palmolive Company,
(iii) Rinsing and drying forearm,
(iv) Wetting the forearm with warm running water,
(v) Using a 1ml syringe with the composition to be tested, applying 0.5 ml of the composition onto the center of the wetted side,
(vi) Lathering the test site for 15 seconds and then leaving the lather on the test site for 30 seconds,
(vii) Rinsing the site under warm, running water for 15 seconds by just allowing the water to flow over the forearm,
(viii) Patting dry with a paper towel the back of the forearm followed by the area of application (the volar forearm),
(ix) Using the same paper towel as in (viii), applying 5 strokes across the application area (volar forearm) to simulate toweling-off effect.

The skin gloss is measured with the Samba Arm System manufactured and marketed by Bossa Nova technologies, LLC (Venice, CA). The panelist inserts her forearm through the instrument opening. A source of polarized light is shone, and the polarized reflection is captured by the Samba polarization camera and translated into gloss by the instrument software. The instrument is used as supplied. There are no settings to adjust. Measurements are taken on the forearm after drying in step (iii) to obtain a background measurement. The forearm is measured again after step (ix). The background measurement is subtracted from the after measurement to show the effect of deposition.

The table below shows the gloss results for the body washes.

| Composition | Gloss |
|---|---|
| Control | 2.8 |
| Untreated mica/titanium dioxide | 2.0 |
| A - mica/titanium dioxide with polyquaternium-67 | 6 |
| B - mica/titanium dioxide first coated with polyquaternium-67 and then coated with isopropyl titanium triisostearate | 7.5 |
| C - mica/titanium dioxide first coated with isopropyl titanium triisostearate and then coated with polyquaternium-67 | 9.5 |

The results show that the surface treated mica/titanium dioxide particles increase the gloss of skin. It is theorized that the surface treated mica/titanium dioxide is being retained on skin because of the surface treatment. The mica/titanium dioxide particles then provide additional reflectance to increase the gloss.

The performance can also evaluated by the panelists who self asses their forearm skin after application of a body wash containing the particles. Panelists are asked the following questions to describe their observations:
(i) How shiny does your skin look? (5 point scale)
   1=not at all shiny, 2=slightly shiny, 3=somewhat shiny, 4=very shiny, extremely shiny
(ii) Is the amount of shine on your skin ... (5 point scale)
   1=not nearly shiny enough. 2=not quite shiny, 3=just about right, 4=somewhat too shiny, 5=much too shiny
(iii) Does you skin look like it sparkles? (3 point scale)
   1=does not sparkle at all, 2=notice a few sparkles on my skin, 3=notice lots of sparkles on my skin
(iv) Brightens the skin (5 point scale)
   1=does not describe at all, 2=does not describe very well, 3=describes somewhat, 4=describes very well, 5=describes completely
(v) Leaves skin looking radiant (5 point scale)
   1=does not describe at all, 2=does not describe very well, 3=describes somewhat, 4=describes very well, 5=describes completely

The body washes described below are prepared by mixing the ingredients. The untreated sample used mica/titanium dioxide with no coating. Each of the other examples include the mica/titanium dioxide particle that is first coated with isopropyl titanium triisostearate and then coated with polyquaternium-67. The base is the same base from above. In the table below, the amounts are based on the overall weight of the material.

| Material | Untreated | D | E | F | G | H | I |
|---|---|---|---|---|---|---|---|
| Base | 98.2 | 98.2 | 93.6 | 92.1 | 95.1 | 93.95 | 90.95 |
| Pigment | 0.8 | 0.8 | 0.8 | 0.8 | 0.8 | 0.8 | 0.8 |
| Sunflower Oil | 0 | 0 | 4 | 4 | 1 | 1 | 4 |
| Petrolatum | 0 | 0 | 0 | 1.5 | 1.5 | 1.5 | 1.5 |
| Guar hydroxypropyltrimonium chloride | 0 | 0 | 0.6 | 0.6 | 0.6 | 0.6 | 0.6 |
| Stepan-Mild™ PCL sodium methyl-2 sulfolaurate and disodium 2-sulfolaurate and sodium lauryl sulfoacetate blend | 0 | 0 | 0 | 0 | 0 | 1.15 | 1.15 |
| Perfume | 1 | 1 | 1 | 1 | 1 | 1 | 1 |
| Total | 100 | 100 | 100 | 100 | 100 | 100 | 100 |
| Number of panelists evaluating | 7 | 7 | 7 | 6 | 6 | 6 | 6 |

The above compositions were evaluated by panelists using the ratings described above. A control using 7 panelists was conducted using commercially available Irish Spring™ original body wash from Colgate-Palmolive Company, which did not contain any pigment particles. The table below shows the average rating given by the panelists.

| Evaluation | Control | Untreated | D | E | F | G | H | I |
|---|---|---|---|---|---|---|---|---|
| How shiny does you skin look? | 1 | 2 | 2.5 | 3.86 | 3.17 | 3.5 | 3.5 | 3.17 |
| Is the amount of shine on your skin ... | 1.29 | 1.86 | 2.33 | 2.86 | 2.83 | 2.83 | 3.33 | 3 |
| Does you skin look like it sparkles? | 1 | 1.86 | 1.83 | 2.43 | 2.33 | 2.42 | 2.83 | 2.5 |
| Brightens the skin | 1.57 | 2.71 | 3.00 | 4.29 | 3.33 | 3.67 | 4.17 | 2.83 |
| Leaves the skin looking radiant | 1.57 | 2.57 | 3.17 | 3.86 | 3.5 | 3.33 | 3.83 | 3.33 |

The table shows that particles made according to the invention impart higher shine and radiance compared to body washes with no particles and body washes with particles with no surface treatments.

## Claims

1. A skin cleansing composition comprising a pigment particle that is pre-coated with Polyquaternium-67, wherein the Polyquaternium-67 is present in an amount of 0.1 to 5 wt% based on the total weight of the pigment particle, and wherein the particle is at least one material chosen from mica/titanium dioxide, mica/iron oxide, and mica.

2. The composition of claim 1, wherein the particle is mica/titanium dioxide.

3. The composition of claim 1, wherein the pigment particle further comprises isopropyl titanium triisostearate.

4. The composition of claim 1 further comprising a surfactant.

5. The composition of claim 1, wherein the composition is a body wash.

6. The composition of claim 1 further comprising at least one material chosen from an oil and petrolatum.

7. The composition of claim 1, wherein the Polyquaternium-67 is present in an amount of 0.2 to 5 wt% based on the total weight of the pigment particle.

8. The composition of claim 1, wherein the Polyquaternium-67 is present in an amount of 0.3 to 5 wt% based on the total weight of the pigment particle.

9. A method of increasing gloss on skin comprising, applying the composition of claim 1 to the skin and rinsing the composition from the skin.

10. The method of claim 9, wherein the pigment particle is mica/titanium dioxide.

11. The method of claim 9, wherein the composition further comprises a surfactant.

12. The method of claim 9, wherein the composition further comprises at least one material chosen from an oil and petrolatum.

## Patentansprüche

1. Hautreinigungszusammensetzung, umfassend ein Pigmentpartikel, welches mit Polyquaternium-67 vorbeschichtet ist, wobei das Polyquaternium-67 in einer Menge von 0.1 bis 5 Gew.%, basierend auf dem Gesamtgewicht des Pigment-Partikels vorliegt, und wobei das Partikel mindestens ein Material ist, ausgewählt aus Glimmer/Titandioxid, Glimmer/Eisenoxid, und Glimmer.

2. Zusammensetzung nach Anspruch 1, wobei das Partikel Glimmer/Titandioxid ist.

3. Zusammensetzung nach Anspruch 1, wobei das Pigmentpartikel des weiteren Isopropyltitantriisostearat umfasst.

4. Zusammensetzung nach Anspruch 1, des Weiteren umfassend einen oberflächenaktives Mittel.

5. Zusammensetzung nach Anspruch 1, wobei die Zusammensetzung eine Waschlotion ist.

6. Zusammensetzung nach Anspruch 1, des Weiteren ein aus Öl und Vaseline ausgewähltes Material umfassend.

7. Zusammensetzung nach Anspruch 1, wobei das Polyquaternium-67 in einer Menge von 0.2 bis 5 Gew.%, basierend auf dem Gesamtgewicht des Pigmentpartikels, vorliegt.

8. Zusammensetzung nach Anspruch 1, wobei das Polyquaternium-67 in einer Menge von 0.3 bis 5 Gew.%, basierend auf dem Gesamtgewicht des Pigmentpartikels, vorliegt.

9. Verfahren zum Erhöhen von Glanz der Haut, umfassend Anwenden der Zusammensetzung gemäß Anspruch 1 auf die Haut und Abspülen der Zusammensetzung von der Haut.

10. Verfahren nach Anspruch 9, wobei das Pigmentpartikel Glimmer/Titandioxid ist.

11. Verfahren nach Anspruch 9, wobei die Zusammensetzung des Weiteren ein oberflächenaktives Mittel umfasst.

12. Verfahren nach Anspruch 9, wobei die Zusammensetzung des Weiteren ein aus Öl und Vaseline ausgewähltes Material umfasst.

## Revendications

1. Composition nettoyante pour la peau comprenant une particule de pigment qui est pré-enduite avec du Polyquaternium-67, dans laquelle le Polyquaternium-67 est présent en une quantité de 0,1 à 5 % en poids par rapport au poids total de la particule de pigment, et dans laquelle la particule est au moins un matériau choisi parmi le mica/dioxyde de titane, le mica/oxyde de fer et le mica.

2. Composition selon la revendication 1, dans laquelle la particule est du mica/dioxyde de titane.

3. Composition selon la revendication 1, dans laquelle la particule de pigment comprend en outre du triisostéarate d'isopropyltitane.

4. Composition selon la revendication 1, comprenant en outre un tensioactif.

5. Composition selon la revendication 1, dans laquelle la composition est un produit lavant pour le corps.

6. Composition selon la revendication 1, comprenant en outre au moins un matériau choisi parmi une huile et du pétrolatum.

7. Composition selon la revendication 1, dans laquelle le Polyquaternium-67 est présent en une quantité de 0,2 à 5 % en poids par rapport au poids total de la particule de pigment.

8. Composition selon la revendication 1, dans laquelle le Polyquaternium-67 est présent en une quantité de 0,3 à 5 % en poids par rapport au poids total de la particule de pigment.

9. Procédé permettant d'augmenter le brillant sur la peau comprenant, l'application de la composition selon la revendication 1 sur la peau et le rinçage de la composition de la peau.

10. Procédé selon la revendication 9, dans lequel la particule de pigment est du mica/dioxyde de titane.

11. Procédé selon la revendication 9, dans lequel la composition comprend en outre un tensioactif.

12. Procédé selon la revendication 9, dans lequel la composition comprend en outre au moins un matériau choisi parmi une huile et du pétrolatum.
